Europäisches Patentamt

European Patent Office

Office européen des brevets

(19)

(11) **EP 0 924 517 A1**

(12) **EUROPEAN PATENT APPLICATION**

(43) Date of publication:
**23.06.1999 Bulletin 1999/25**

(51) Int Cl.6: **G01N 31/00**, G01N 31/12,
G01N 33/00

(21) Application number: **98204333.3**

(22) Date of filing: **18.12.1998**

| (84) | Designated Contracting States:<br>**AT BE CH CY DE DK ES FI FR GB GR IE IT LI LU MC NL PT SE**<br>Designated Extension States:<br>**AL LT LV MK RO SI** | (72) | Inventors:<br>• **Sprinkhuizen, Quirinus Adrianus**<br>  **2521 SW 'S-Gravenhage (NL)**<br>• **Kampert, Hendrikus Gerardus Adrianus**<br>  **3438 RM Nieuwegein (NL)**<br>• **Neelemaat, Johannes Adriaan**<br>  **1444 EN Purmerend (NL)** |
| --- | --- | --- | --- |
| (30) | Priority: **19.12.1997 NL 1007860** | | |
| (71) | Applicant: **Euroglas B.V.**<br>**2627 BC Delft (NL)** | (74) | Representative: **de Bruijn, Leendert C. et al**<br>**Nederlandsch Octrooibureau**<br>**P.O. Box 29720**<br>**2502 LS Den Haag (NL)** |

(54) **Method and apparatus for analysing a sample**

(57) The present invention relates to a method and an apparatus for analysing a sample, wherein said sample is passed in vapour or gas form, with the aid of a reduced pressure or a capillary, via a feed line to a reaction or measurement chamber, wherein the flow of said sample through said feed line is controlled with the aid of an expansion facility such that essentially no sample is lost and that an essentially constant feed of vapour or gas takes place.

fig-2

EP 0 924 517 A1

## Description

The present invention relates to a method for analysing a sample, wherein the sample is passed in vapour or gas form, with the aid of a reduced pressure or a capillary, via a feed line to a reaction or measurement chamber.

In the prior art it is known to analyse the composition of samples with the aid of apparatus or a method with which the sample is passed in vapour or gas form, with the aid of a reduced pressure or a capillary, to a reaction or measurement chamber. In said reaction or measurement chamber, for example, the presence of one or more elements in the sample is determined.

In order to be able to analyse liquid and solid samples with the aid of such a method, said samples are usually vaporised and/or combusted.

The above method can, for example, be used to determine the nitrogen content in samples. In such a case the nitrogen oxide (NO) is brought into the excited state in the reaction or measurement chamber with the aid of ozone. The light emitted on decay of said excited nitrogen oxide is measured using a photomultiplier tube (PMT).

$$NO + O_3 \rightarrow NO_2^{\cdot} + O_2$$

$$NO_2^{\cdot} \rightarrow NO_2 + h\nu$$

If this measurement method is to function well, the nitrogen present must as far as possible be converted into the highly stable NO before the sample is fed to the reaction or measurement chamber. In order to achieve this, the sample in vapour or gas form is usually fed through a furnace in which oxygen is supplied to the sample and in which the sample is passed over a platinum catalyst.

The method described is used, inter alia, to determine the nitrogen content in drinking water, groundwater, effluent, sludge, sediments and numerous hydrocarbons and other biological and chemical products. Usually the nitrogen content of a relatively small sample, having a typical volume of approximately 50 $\mu$l or approximately 50 mg in weight, is determined. It is therefore extremely important that analysis of the sample is carried out with the greatest possible accuracy.

A first significant disadvantage of the method according to the prior art is that, especially when analysing hydrocarbons, the feed rate of the sample is, inter alia, dependent on the expansion of the sample in the combustion furnace. Especially when analysing hydrocarbons, this expansion will be so great that it is not the reduced pressure intended for the feed, or the capillary intended for the feed, which determines the feed rate to the reaction or measurement chamber, but that the expansion itself is responsible for the feed rate of the sample to the reaction or measurement chamber. As a consequence of this uncontrolled feed rate of the sample to the reaction or measurement chamber, the analysis carried out using the method according to the prior art is relatively inaccurate.

A first aim of the present invention is therefore to provide a method for analysing a sample, wherein the sample in vapour or gas form is passed, with the aid of a reduced pressure or a capillary, via a feed line to a reaction or measurement chamber, with which said disadvantage according to the prior art will not arise.

In the present invention said aim is achieved in that the flow of the sample through the feed line is controlled with the aid of an expansion facility such that essentially no sample is lost and in that an essentially constant feed of vapour or gas takes place.

With this procedure it is advantageous that the flow rate of the sample through the feed line is 100 to 3000 ml/min, preferably 400 to 1500 ml/min and particularly advantageously ± 800 ml/min. Furthermore, with this procedure approximately 400 ml/min of ambient air or inert gas is drawn in, so that total flow to the reaction or measurement chamber is, for example, 1200 ml/min.

The effect of these measures is that, irrespective of the expansion of the sample in the combustion furnace or the feed lines, the feed rate of the sample to the reaction or measurement chamber is virtually constant, whilst no sample is lost when feeding the sample to the reaction or measurement chamber. This means that the actual determination of the presence of components in the sample, in the reaction or measurement chamber, can take place with a much greater accuracy.

A second significant disadvantage of the method according to the prior art is that the flow rate through the combustion furnace, and thus through the catalyst, is also partly dependent on the expansion of the sample in said combustion furnace. When, for example, the nitrogen content is determined with the aid of the method according to the prior art, the expansion in the combustion furnace can, especially in the case of hydrocarbons, be so great that the reaction process for the conversion of nitrogen to NO does not go to completion. Especially in the case of hydrocarbons, alkenes (olefins) can be formed if the combustion does not go to completion. These alkenes also react with the ozone in the reaction or measurement chamber. The light emitted during this reaction is in the same wavelength region as the light which is measured on decay of excited nitrogen oxide. Consequently, the presence of alkenes in the reaction or measurement chamber interferes with the determination of nitrogen oxide present. If the analytical apparatus according to the present invention is to function properly it is therefore necessary that all hydrocarbons present are completely combusted.

A second aim of the present invention is, therefore, to provide a method with which heating of the sample in the combustion furnace, the supply of oxygen to said sample in said combustion furnace and passing said sample through the catalyst are carried out in such a way that the abovementioned problems according to the prior art will not arise.

In the present invention said aim is achieved in that said method comprises:

a) if required, vaporising the sample in a chamber at a high temperature in a stream of inert gas,

b) supplying oxygen to the gas stream formed in step a),

c) passing the gas stream from step b) over a catalyst,

d) passing the gas stream, after leaving the catalyst, into a chamber in the direction of the wall of said chamber, a second stream of oxygen being supplied to the gas stream, the direction of flow of said second stream of oxygen being counter to the outflow direction from the catalyst, and

e) discharging the gas stream to analytical apparatus located further downstream, wherein, on discharge of the gas stream, a third stream of oxygen is introduced into the gas stream, the direction of flow of said third stream of oxygen being essentially counter to the discharge direction of the gas stream.

With this method it is advantageous that steps a), b), c) and d) are carried out at a temperature of at least 850 °C and step e) is carried out at a temperature of at least 1000 °C.

The effect of these measures is that the expansion of the sample, as a result of the controlled supply of the oxygen and the inert gas to the sample before the sample is passed through the catalyst, is relatively restricted. Moreover, in the method according to the present invention the sample is fed towards the relatively hot wall of the chamber downstream of the catalyst. The reaction processes will be further intensified as a result. By, moreover, introducing a third stream of oxygen into the gas stream, the reaction processes will be further assisted by the turbulence resulting from this.

The present invention further relates to an apparatus for analysing a sample, comprising a reaction or measurement chamber and a feed line, for the sample, which is connected to said reaction or measurement chamber, wherein said apparatus is provided with a capillary and/or means providing a reduced pressure, with the aid of which said gaseous sample is passed towards said reaction or measurement chamber.

The apparatus according to the present invention is characterised in that said feed line is connected to an expansion facility in which essentially atmospheric pressure prevails.

The apparatus according to the present invention is in particular suitable for carrying out the abovementioned method according to the present invention.

Research carried out by the Applicant has shown that numerous known technical solutions are not usable in the apparatus according to the present invention. Because, certainly in the case of hydrocarbons, the expansion of the sample in the combustion furnace proceeds very rapidly and very violently, it is, for example, not possible to provide the feed line to the reaction or measurement chamber with an expansion vessel having, for example, a piston therein. This is because it is not possible, with the aid of such apparatus, to respond quickly enough to changes in pressure in the feed line and to keep the flow rate in the feed line constant. The said research showed, surprisingly, that an expansion facility in which essentially atmospheric pressure prevails can, however, be used to keep the flow rate through the feed line constant.

The apparatus according to the present invention is further improved in that said expansion facility is in open communication with the environment, or with a source of essentially unpressurised gas.

The effect of this measure is that, as a result, adequate control of the feed rate to the reaction or measurement chamber is achieved. Moreover, research has shown that, despite the open communication which the expansion facility has with the environment, no sample flowed out of the expansion facility and was lost during analysis.

In one possible embodiment, the expansion facility of the apparatus according to the present invention is formed by one or more hoses or tubes, the one end of which is connected to said feed line and the other end of which is in open communication with the environment or with a source of essentially unpressurised inert gas.

With this arrangement it is advantageous that said expansion facility is in communication with the environment via a syphon. The effect of this measure is that an expansion facility can be formed in a very simple manner. By, furthermore, making said hoses or tubes flexible, said hoses or tubes can simply be draped in the apparatus according to the present invention.

In order to ensure adequate reaction processes in the combustion furnace, the present invention also provides an apparatus which comprises:

- a first furnace zone, wherein said furnace zone comprises
- a first combustion chamber, provided at the first end thereof with an opening for the introduction or supply of a sample, wherein said combustion chamber is provided close to said first end with a connection for a line for supplying an inert gas to said combustion chamber, and wherein the second end of said combustion chamber forms an outflow opening, which is connected to the inflow opening of a catalyst chamber, wherein said first combustion chamber is provided close to said second end thereof with a connection for a line for supplying oxygen to said combustion chamber,
- an essentially cylindrical catalyst chamber, which is connected, by the first end thereof, to the outflow opening of said first combustion chamber, and the second end of which forms an outflow opening which is connected to a second combustion chamber, wherein said second combustion chamber is provided, close to the first end thereof, with a connection for a line for the supply of oxygen and the second end of said combustion chamber forms an outflow opening,

- a second furnace zone, wherein said second furnace zone comprises
- a third combustion chamber provided, at the first end thereof, with an inflow opening which is connected to the outflow opening from the second combustion chamber of the first furnace zone and the second end of which forms an outflow opening.

With this arrangement, the apparatus according to the present invention is characterised in that the outflow opening from the catalyst chamber communicates with the aid of one or more channels with the second combustion chamber, wherein the channels are positioned such that the direction of flow through the channels is counter to the direction of flow through the catalyst chamber and wherein the channel or channels open into an outflow opening which is shaped such that a gas stream is forced through the outflow opening in the direction of the wall of the second combustion chamber.

The advantage of the said apparatus according to the present invention is that the flow in the chamber downstream of the catalyst is highly turbulent. Moreover, on leaving the catalyst, the sample is forced in the direction of the relatively hot wall of said chamber. As a result of these two effects, the reaction processes are intensified.

The apparatus according to the present invention is further improved in that the third combustion chamber is provided close to the outflow opening thereof with a connection for a line for supplying oxygen, wherein said connection is shaped such that said stream of oxygen is directed from the outflow opening of the combustion chamber towards the opposite end thereof.

The effect of this measure is that, as a result of the supply of the stream of oxygen, the turbulence in the gas stream is further increased. The reaction processes will be further intensified as a result.

In an advantageous embodiment, the outflow opening from the second combustion chamber is connected to an essentially spiral-shaped channel, wherein the outflow opening thereof is connected to an input channel, the one end of which is connected to the line for supplying oxygen in the third combustion chamber, and the other end opens into the third combustion chamber.

The effect of this measure is that there is very good mixing of the sample that is fed from the second combustion chamber to the third combustion chamber and the oxygen that is supplied to the latter combustion chamber.

The present invention will be described below on the basis of a non-limiting illustrative embodiment, with reference to the appended drawings, in which:

Figure 1 shows a cross-section of the combustion furnace for the sample according to the present invention.

Figure 2 shows, diagrammatically, an analytical apparatus in which the combustion furnace and the expansion facility according to figure 1 are shown.

Figure 3 shows, diagrammatically, a preferred embodiment of an expansion facility according to the present invention.

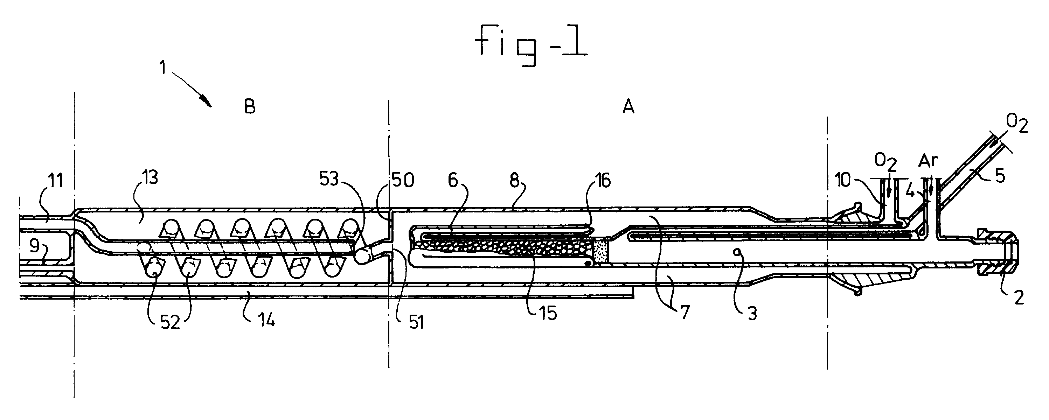

The combustion furnace 1 according to a preferred embodiment of the present invention is shown in Figure 1. The combustion furnace 1 comprises a zone A (on the right in the drawing) and a zone B, which zones are separated from one another by means of a partition 50. Furnace zone A has an opening 2, which, during use, is connected to a system for the introduction of a sample into the first combustion chamber 3. The sample can, for example, be introduced with the aid of a needle or boat (not shown) which is connected to an autosampler.

The temperature prevailing in the furnace zone A differs from that in the furnace zone B. The temperature in zone A is ± 850 °C. The temperature in zone B is ± 1000 °C.

As can be seen in Figure 1, the first combustion chamber 3 is located in furnace zone A. An inert gas, for example argon, is introduced into the first combustion chamber 3 from the feed line 4. The argon stream so introduced will move through the first combustion chamber 3 in the direction of the catalyst 15. Under the influence of the high temperature of 850 °C and the argon stream, solid or liquid sample in the first combustion chamber 3 can vaporise. With the aid of the oxygen line 5, oxygen is blown into the gas stream formed in the first combustion chamber 3 just upstream of the catalyst 15. As a result, the sample is able to combust. The gas stream then moves through the catalyst 15 in the direction of the outflow channels 6. The catalyst 15 is made, for example, of platinum on, for example, aluminium oxide. After leaving the catalyst 15, the gas stream moves through the outflow opening 6 in the direction of the second combustion chamber 7. With this arrangement the outflow openings 16 of the channels 6 are shaped such that the gas stream flows out in the direction of the wall 8 of the second combustion chamber 7. Because the wall 8 is extra hot, the reaction processes in the gas stream are intensified as a result. Two outflow openings 6 are shown in Figure 1. It is also possible to use three, four or more outflow openings 6. Oxygen is fed from a second oxygen feed line 10 to the second combustion chamber 7. The oxygen will mix with the gas stream from the channels 6 and flow together with the latter in the direction of the opening 51 in the partition 50. A spiral-shaped channel 52, which opens into an oxygen line 11, is connected to the opening 51. Additional oxygen is supplied to the gas stream via the line 11. Good mixing between the gas stream and the additional oxygen supplied is achieved by this means. The gas stream leaves the line 52 via the outflow opening 53 and flows through the third combustion chamber 13 in the direction of the discharge channel 9.

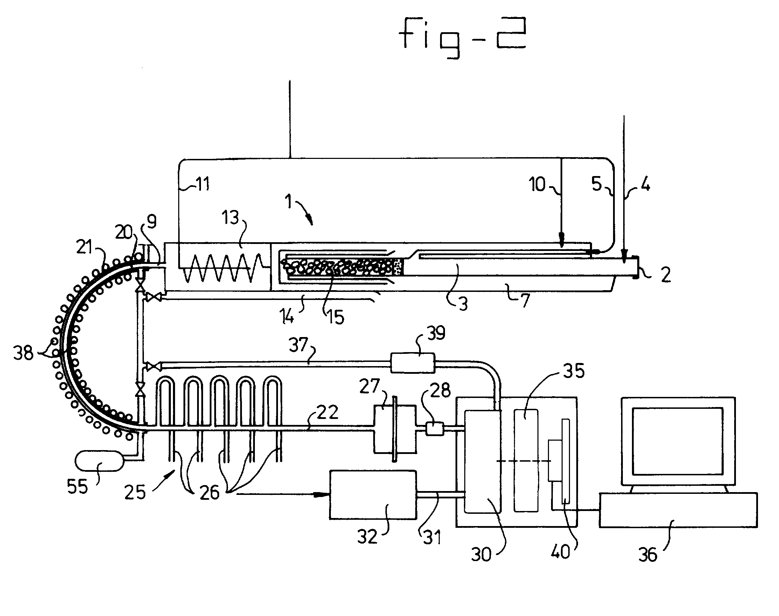

An analytical apparatus according to the present invention can be seen in Figure 2. It can be seen from Figure 2 that the discharge line 9 from the combustion furnace 1 is connected to a line 20, which is provided on the outside with a second line 21. The line 20 is preferably made of perma-pure. Perma-pure is a copolymer which has sulphuric acid groups at the end. The property of this material is that the water from the gas stream flowing through the line 20 is able to discharge through the tube wall. The gas stream is thus "dried" by said perma-pure line. In order to achieve this effect, a dry gas must flow in counter-current over the outside of the perma-pure line. In this context it is important that said counter-current is at most approximately equal in magnitude to the gas stream flowing through the perma-pure line itself. In drawing 2 this counter-current line is indicated by line 21. Furthermore, a heating device 38 is fitted on the outside of line 21 in order to be able to supply heat from the outside to the counter-current line 21 and the perma-pure line 20. After leaving the line 20, the gas stream flows through the line 22, which is provided with an expansion facility 25 according to the present invention. The expansion facility consists of a number of channels, for example hoses, which at the one end are connected to the line 22 and at the other end are in open communication with the environment or a source of inert gas which is virtually unpressurised, that is to say the inert gas is under virtually atmospheric pressure. Normally the apparatus according to the present invention will be used in an area in which atmospheric pressure prevails. This means that atmospheric pressure also prevails in the expansion facility 25. However, if the apparatus according to the present invention is used in an area in which there is sub-atmospheric pressure, a corresponding sub-atmospheric pressure will prevail in the expansion facility. After leaving the line 22, the gas stream will flow through a glass fibre filter 27. A capillary 28 is fitted on the downstream side of the glass fibre filter.

Furthermore, a vacuum pump 39 is fitted in the apparatus. The size of the capillary 28 is chosen such that, in combination with the vacuum pump 39, under a given vacuum a constant flow is supplied to the reaction or measurement chamber 30. In use, the combination of the capillary 28 and the vacuum pump 39 will be chosen such that, in addition to the flow of the sample through the line 22, a certain amount of ambient air or inert gas is also drawn continuously through the channels 26 of the expansion facility 25. In this context it is possible, for example, to choose a flow of 1200 ml per minute to the reaction or measurement chamber 30, said flow being made up of 800 ml per minute sample and 400 ml per minute ambient air or inert gas.

After leaving the filter 27, the sample will flow into the reaction or measurement chamber 30. Line 31, which is connected to ozonator 32, is also connected to the reaction or measurement chamber 30. Oxygen is converted to ozone in said ozonator. The gas present in the reaction or measurement chamber 30 is brought into the excited state with the aid of the ozone. The light emitted is then measured via the optical filter 35 using a photomultiplier tube (PMT) 40. The measured values are then displayed with the aid of display means, for example a PC 36.

After leaving the reaction or measurement chamber 30, the gas flows via the pump 39, with the aid of the line 37, back in the direction of the furnace 1. At this stage the gas can be fed to the line 14, which is positioned directly adjacent to the furnace 1. Since the temperature in the line 14 is relatively high, the harmful elements in the gas stream in the line 14 will be converted under the influence of said high temperature into non-harmful residual products.

After leaving the line 37, the gas stream can, however, also be fed to the counter-current line 21, which runs over the outside of the perma-pure line 20. An advantage of this measure is that the off-gas is utilised in this way and dry gas, which has to be specially supplied in order to be fed through the line 21, can be saved. Since, however, the counter-flow through the line 21 may not be higher than the flow through the line 20, not all of the gas that comes from the line 37 will be able to be discharged through the line 21 in the direction of, for example, the line 14. After leaving the line 21, the gas stream can be discharged to processing means, or can be supplied to the line 14, in which, as has already been pointed out, the harmful products can be converted into non-harmful residual products.

If it is not desired to feed gas issuing from the reaction or measurement chamber 30 through the line 21, dry gas can be supplied from a reservoir to the line 21.

Advantages of the apparatus according to the present invention which is shown in Figure 2 are twofold. Firstly, the conversion of the sample in the combustion furnace 1 according to the present invention is better controlled and more complete, as a result of the effects mentioned in the description of Figure 1. The risk of alkenes in the reaction or measurement chamber 30 which interfere with the determination of the nitrogen oxide present is thus restricted to a minimum.

The expansion facility 25 according to the present invention functions as follows. In normal operation, the feed rate of the gas stream to the reaction or measurement chamber 30 will be determined by the capillary 28 and the vacuum pump 39. If there is substantial expansion of the sample, the pressure in the feed line 22 will rise. As a result there is the risk that no longer only the capillary 28 and the vacuum pump 39, but also the pressure in the line 20 will be determining factors for the feed flow rate to the reaction or measurement chamber 30. However, as a result of the presence of the expansion facility 25, for example formed by the line 26, the gas stream will be able to flow unpressurised in the lines 26. The flow rate through the capillary 28 is consequently not disturbed. If the pressure in the line 22 subsequently falls, the sample flowing in the lines 26 will also be fed to the reaction or measurement chamber 30 with the aid of the capillary 28. Since no sample is lost if the expansion facility 25 is correctly sized, the flow rate to the reaction or measurement chamber 30 will not only be constant but, furthermore, all of the sample introduced into the apparatus will pass into the reaction or measurement chamber 30. As a result of these effects, a very accurate measurement in said reaction or measurement chamber 30 is possible.

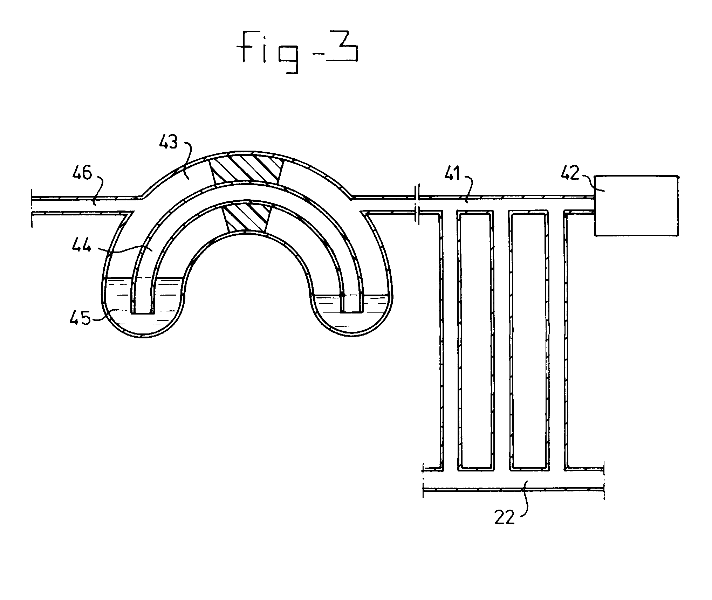

Finally, a possible embodiment of the expansion facility 25 according to the present invention is shown in Figure 3. Figure 3 shows the line 22 through which the sample flows from the perma-pure line in the direction of the reaction or measurement chamber. In the case shown, three channels 26 are fitted to said line 22, which channels 26 merge at the other end thereof into the line 41. The one end of the line 41 is connected to a source of inert gas 42. The other end of the line 41 is connected to a syphon-like construction 43 in which a U-shaped tube 44 is positioned, the end of said U-shaped tube being completely or partially surrounded by a fluid, for example water 45. The syphon-like construction 43 also has an outflow end 46, which is in open communication with the ambient air. The device shown in Figure 3 functions as follows:

If too high a pressure temporarily prevails in the line 22, the sample supplied through the line 22 will flow through the channels 26 in the direction of the line 41. This will cause the gas present in the lines 26 to be propelled in the direction of the syphon; like element 43 and said gas will be able to flow out via the outflow opening 46. The flow through the line 22 itself will consequently not be seriously disrupted even in the event of a temporarily higher pressure.

During normal use, sample can be supplied at, for example, a rate of 800 ml per minute. Ambient air is also supplied through the channels 26 and flows through the syphon-like element and the line 41 in the direction of the channels 26. When the source of inert gas is open, inert gas can also be drawn through the channels 26 in the direction of the line 22. For example, 400 ml per minute are drawn in through the line 41 and the channels 26. The total flow of sample plus added outside air or inert gas is 1200 ml per minute in the given case.

It must be understood that the illustrative embodiment to which reference is made in Figures 1, 2 and 3 serves for illustration only. The invention is not restricted to the example given here, but relates to methods and apparatus according to the appended claims.

**Claims**

1. Method for analysing a sample, wherein the sample is passed in vapour or gas form, with the aid of a reduced pressure or a capillary (28), via a feed line (22) to a reaction or measurement chamber (30), characterised in that the flow of the sample through said feed line (22) is controlled with the aid of an expansion facility (25) such that essentially no sample is lost and in that an essentially constant feed of vapour or gas takes place.

2. Method according to Claim 1, characterised in that the flow rate of the sample through the feed line (22) is 100 to 3000 ml/min, preferably 400 to 1500 ml/min and particularly advantageously ± 750-850 ml/min.

3. Method for analysing a sample according to Claim 1 or 2, characterised in that the following steps are carried out:

   a) if required, vaporising the sample in a chamber (3) at a high temperature in a stream of inert gas,
   b) supplying oxygen to the gas stream formed in step a),
   c) passing the gas stream from step b) over a catalyst (15),
   d) passing the gas stream, after leaving the catalyst (15), into a chamber (7) in the direction of the wall (8) of said chamber (7), a second stream of oxygen being supplied to the gas stream, the direction of flow of said second stream of oxygen being counter to the outflow direction from the catalyst (15), and
   e) discharging the gas stream to analytical apparatus (30, 35, 40) located further downstream, wherein, on discharge of the gas stream, a third stream of oxygen is introduced into the gas stream, the direction of flow of said third stream of oxygen being essentially counter to the discharge direction of the gas stream.

4. Method according to Claim 3, characterised in that steps a), b), c) and d) are carried out at a temperature of at least 850°C and step e) is carried out at a temperature of at least 1000 °C.

5. Apparatus for analysing a sample, comprising a reaction or measurement chamber (30) and a feed line (22), for the sample, which is connected to said reaction or measurement chamber (30), wherein said apparatus is provided with a capillary (28) and/or means for providing a reduced pressure, with the aid of which said gaseous sample is passed towards said reaction or measurement chamber (30), characterised in that said feed line (22) is connected to an expansion facility (25) in which essentially atmospheric pressure prevails.

6. Apparatus according to Claim 5, characterised in that said expansion facility (25) is in open communication with the environment, or with a source (42) of essentially unpressurised gas.

7. Apparatus according to Claim 5 or 6, characterised in that said expansion facility (25) is formed by one or more hoses or tubes (26), the one end of which is connected to said feed line (22) and the other end of which is in open communication with the environment or with a source (42) of essentially unpressurised inert gas.

8. Apparatus according to Claim 7, characterised in that said expansion facility (25) is in communication with the environment via a syphon (43).

9. Apparatus according to Claim 5, 6, 7 or 8, wherein said apparatus comprises:

- a first furnace zone (A), wherein said furnace zone (A) comprises
- a first combustion chamber (3), provided at the first end thereof with an opening (2) for the introduction or supply of a sample, wherein said combustion chamber is provided close to said first end with a connection for a line (4) for supplying an inert gas to said combustion chamber (3), and wherein the second end of said combustion chamber (3) forms an outflow opening, which is connected to the inflow opening of a catalyst chamber (15), wherein said first combustion chamber is provided close to said second end thereof with a connection for a line (5) for supplying oxygen to said combustion chamber (3),
- an essentially cylindrical catalyst chamber (15), which is connected, by the first end thereof, to the outflow opening of said first combustion chamber (3), and the second end of which forms an outflow opening which is connected to a second combustion chamber (7), wherein said second combustion chamber (7) is provided, close to the first end thereof, with a connection for a line (10) for the supply of oxygen and the second end of said combustion chamber (7) forms an outflow opening (51),
- a second furnace zone (B), wherein said second furnace zone (B) comprises
- a third combustion chamber (13) provided, at the first end thereof, with an inflow opening (51) which is connected to the outflow opening (51) from the second combustion chamber (7) of the first furnace zone (A) and the second end of which forms an outflow opening (9), characterised in that
the outflow opening from the catalyst chamber (15) communicates with the aid of one or more channels (6) with the second combustion chamber (7), wherein the channels (6) are positioned such that the direction of flow through the channels (6) is counter to the direction of flow through the catalyst chamber (15) and wherein the channel or channels (6) open into an outflow opening (16) which is shaped such that a gas stream is forced through the outflow opening (16) in the direction of the wall (8) of the second combustion chamber (7).

10. Apparatus according to one of Claims 5 - 9, characterised in that the third combustion chamber (13) is provided close to the outflow opening (9) thereof with a connection for a line (11) for supplying oxygen, wherein said connection is shaped such that said stream of oxygen is directed from the outflow opening (9) of the third combustion chamber (13) towards the opposite end thereof.

11. Apparatus according to one of Claims 5 - 10, characterised in that the outflow opening (51) from the second combustion chamber (7) is connected to an essentially spiral-shaped channel (52), wherein the outflow opening thereof is connected to an input channel, the one end of which is connected to the line (11) for supplying oxygen in the third combustion chamber (13), and the other end thereof is in open communication with the third combustion chamber (13).

# fig-1

EP 0 924 517 A1

Fig-2

Fig-3

**European Patent Office**

**EUROPEAN SEARCH REPORT**

Application Number

EP 98 20 4333

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | US 5 616 822 A (GRIFFITHS ET AL.) 1 April 1997 * column 3, line 63 - column 4, line 9; figure 1 * | 1,2,5 | G01N31/00 G01N31/12 G01N33/00 |
| Y | EP 0 437 405 A (SOCIÉTÉ NATIONALE ELF AQUITAINE) 17 July 1991 * page 4, line 6 - line 46; figures 1-3 * | 1,2,5 | |
| A | WO 94 07134 A (ROSEMOUNT ANALYTICAL INC.) 31 March 1994 * the whole document * | 1-11 | |
| A | DE 35 38 778 A (W.C. HERAEUS GMBH) 7 May 1987 * the whole document * | 1-11 | |
| A | US 4 333 735 A (HARDY ET AL.) 8 June 1982 * the whole document * | 1-11 | |

**TECHNICAL FIELDS SEARCHED (Int.Cl.6)**

G01N

The present search report has been drawn up for all claims

| Place of search | Date of completion of the search | Examiner |
|---|---|---|
| THE HAGUE | 16 February 1999 | Bosma, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**  EP 98 20 4333

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-1999

| Patent document cited in search report | | Publication date | Patent family member(s) | | Publication date |
|---|---|---|---|---|---|
| US 5616822 | A | 01-04-1997 | AU | 674900 B | 16-01-1997 |
| | | | AU | 5430494 A | 08-06-1994 |
| | | | CA | 2149911 A | 26-05-1994 |
| | | | DE | 69312507 D | 28-08-1997 |
| | | | DE | 69312507 T | 06-11-1997 |
| | | | DK | 670039 T | 22-09-1997 |
| | | | EP | 0670039 A | 06-09-1995 |
| | | | FI | 952432 A | 18-07-1995 |
| | | | WO | 9411732 A | 26-05-1994 |
| | | | GB | 2289127 A,B | 08-11-1995 |
| | | | JP | 8503073 T | 02-04-1996 |
| | | | NO | 951960 A | 19-07-1995 |
| | | | NZ | 257621 A | 27-02-1996 |
| EP 437405 | A | 17-07-1991 | FR | 2657166 A | 19-07-1991 |
| | | | CA | 2034066 A | 13-07-1991 |
| | | | US | 5185268 A | 09-02-1993 |
| WO 9407134 | A | 31-03-1994 | EP | 0660927 A | 05-07-1995 |
| | | | JP | 8501393 T | 13-02-1996 |
| DE 3538778 | A | 07-05-1987 | NONE | | |
| US 4333735 | A | 08-06-1982 | NONE | | |